# EUROPEAN PATENT SPECIFICATION

(11) **EP 1 491 199 B1**
(45) Date of publication and mention of the grant of the patent: **10.09.2008**
(21) Application number: 04386021.2
(22) Date of filing: 17.06.2004
(51) Int. Cl.: A61K 31/66, A61K 9/20

(54) **Pharmaceutical compositions of alendronate sodium trihydrate and process for the preparation thereof**
Pharmazeutische Zubereitung von Alendronat Natrium Trihydrat und Verfahren deren Herstellung
cOMPOSITION pharmaceutique d'Alendronate de Sodium Trihydrate et procédé de préparation de celle-ci& x9;

(30) Priority: 25.06.2003 GR 2003100275
(43) Date of publication of application: 29.12.2004
(73) Proprietor: Pharmanel Pharmaceutical, Commercial and Industrial S.A., Gerakas, Attika (GR)
(72) Inventor: Peponaki, Charikleia, Agios Dimitrios, Attika (GR)
(74) Representative: Masoulas, Athanasios V.

(56) References cited:
- WO-A-97/44017

## Description

### TECHNICAL FIELD OF THE INVENTION

The present invention relates to improved dosage forms such as tablets and in particular to a tablet formulation comprising a therapeutically effective quantity of alendronate sodium trihydrate (4-amino-1-hydroxybutylidene-1,1-bisphosphonic acid sodium salt trihydrate) containing Spray Dried Maltose in combination with Crospovidone and Calcium Stearate and the preparation thereof by a dry granulation process.

### BACKGROUND OF THE INVENTION

Alendronic acid (4-amino-1-hydroxybutylidene-1,1-bisphosphonic acid) is a known compound disclosed in Izv. Akad. Nauk. SSR, Ser. Khim. 2: 433-437 (1978). Methods for the industrial preparation of alendronate sodium trihydrate may be found, inter alia, in DE 3016289 (1980, Henkel KGAA, Germany - & US-A-4 407 761), in IT 1196315 (1984, Gentili IST spa, Italy - & US-A-4 705 651), in EP 402152 (1990, Merck & Co, U.S.A.) and in several other patents. The main object in all these documents is to minimize the concentration of PCl₃ impurities in the final product,which PCl₃ has been used as phosphonylating reagent in the synthetic process. This reagent is not used in the process disclosed in ES 9901806 (1999, Medichem S.A., Spain - & EP 1 205 484), thus providing a decisive solution to the problem.

Due to their low bioavailability, the oral dosage forms of the alendronic acid have to be taken on an empty stomach (in the morning) with a glass of water, and food intake or lying down it is not allowed for at least 30 minutes after administration. In combination with some upper gastrointestinal tract adverse effects, these dosage forms often cause bad compliance in particular by elderly patients. However, several clinical studies in animals since 1991 and later on, have demonstrated that the administration of higher (cumulative) doses taken less frequently achieve the same therapeutic effects with those obtained by administration on a daily basis (J.G. Seedor et al, J. Bone. Miner. Res. 6, 339-346, 1991 ; R. Balena et al, J. Clin. Invest. 92, 2577-2586, 1993 ; G.A. Rodan et al, Osteoporos. Int. Suppl. 3, S7-12, 1993 ; B.J. Gertz et al, Osteoporos. Int. Suppl. 3, S13-16, 1993 ; S.A. Khan et al, J. Bone Miner. Res. 12, 1700-1707, 1997 ). Thus, taking also into consideration that high doses of alendronic acid in human beings were tolerable (e.g. 40mg daily for 6 months in Paget's disease), the substitution of the 10mg alendronic acid daily dose by a 70mg weekly dose in osteoporosis patients has gradually gained ground since 1996 and thereafter (see article in Lunar News I, April 1996, Lunar Corp.).

Alendronic acid and a number of other bisphosphonic acid derivatives, such as clodronic, pamidronic, risedronic acids and the salts thereof, are known to be active in calcium and phosphate metabolism mediated disorders. Effects of bisphosphonic acids on the skeletal system have been known for many years, e.g. the retardation of chronic arthritis progression is disclosed in Brit. J. Pharmacology 21,127 (1963) and the inhibition of osteo-resorption in rats is described in Acta. Endocrinol. 76,613 (1976).

Alendronate sodium trihydrate is the orally bioavailable form of alendronic acid acting as a specific inhibitor of osteoclast mediated bone resorption, and is indicated for the treatment of numerous diseases such as osteoporosis, postmenopausal osteoporosis, osteodystrophy, Paget's disease, myositis ossificans, Bechterew's disease, malignant hypercalcemia, metastatic bone disease, periodontal disease, cholelithiasis, nephrolithiasis, urolithiasis, urinary calculus, sclerosis, arthritis, bursitis, neuritis and tetany.

Various methods are already known for the industrial preparation of oral dosage forms comprising alendronic acid as an active ingredient, and lactose as a preferred diluent due to its known excellent properties of solubility, compressibility etc.. However, the prior art has encountered substantial difficulties in the production of the oral solid formulations of a desirable stability due to the fact that lactose in contact with molecules bearing a primary amino group (such as alendronic acid) is known to undergo browning (Maillard) reactions, thus accelerating the degradation of the active ingredient.

Moreover, EP 0 690 719 (& WO 94/1220) of Merck discloses a direct compression (dry mix) process for the tablet formulation, which requires only blending of the ingredients without granulation or addition of water prior to compression and wherein the mixture comprises an active ingredient with a diluent selected from special grades of anhydrous lactose or hydrous fast flow lactose, a dry binder such as microcrystalline cellulose, a disintegrant such as croscarmellose sodium and magnesium stearate as a lubricant. Comparative tests at 40° C and 75% relative humidity have indicated an increased stability of the direct compression product over the granulated product, however, the problem has still not been quite solved, as indicated by the fact that the same company (Merck) has later proposed an enteric coating for the direct compression tablets (WO 95/08331), as well as a process of wet granulation (WO 95/29679, EP 0 756 484) thus disputing the extent of the advantages provided by the direct compression process. Furthermore, an alternative manufacturing process involving wet granulation of excipients followed by direct compression of the dry granules with alendronic acid is disclosed in PL 340087 ( & WO 01/85176).

### SUMMARY OF THE INVENTION

It is, therefore, an object of the present invention to provide an improved tablet formulation containing alendronic acid or the salt thereof as an active ingredient, which overcomes the deficiencies of the prior art and avoids the degradation of the active ingredient.

A further aspect of the present invention is to provide a process for the preparation of pharmaceutical compositions containing alendronic acid or the salt thereof as an active ingredient with acceptable stability and avoiding the browning caused by the Mailard's reaction.

In accordance with the above objects of the present invention, a pharmaceutical composition is provided comprising from about 1 to 50% by weight of Alendronate Sodium Trihydrate as an active ingredient, and from about 50 to 99 % by weight of excipients, wherein said excipients comprising a basic diluent selected from Spray Dried Maltose, a disintegrant and a lubricant.

According to another embodiment of the present invention , a process for the preparation of solid dosage forms (such as tablets) containing as an active ingredient alendronic acid or the salt thereof is provided, which comprises:
- Weighing the required quantities of the active ingredient and all excipients;
- Forming a mixture by mixing the total quantity of the active ingredient with 50% of the quantity of a diluent selected from Spray Dried Maltose for 10 minutes;
- Slugging the above mixture;
- Screening the slugged mixture through a screen of 8 mesh, and subsequently through a screen of 20 mesh;
- Adding to the screened mixture the remaining 50% of the quantity of the Spray Dried Maltose , and the total quantities of a disintegrant and a lubricant and mixing for 30 minutes, and
- Tabletting, by compressing the resulting lubricated mixture into a desired tablet form.

Further preferred embodiments of the present invention are defined in dependent claims 2 to 7 and 9 to 11.

Other objects and advantages of the present invention will become apparent to those skilled in the art in view of the following detailed description.

### BRIEF DESCRIPTION OF THE DRAWINGS

Fig. 1 shows a dissolution profile of a 10mg tablet according to the present invention.
Fig. 2 shows a dissolution profile of a 70mg tablet according to the present invention.

### DETAILED DESRIPTION OF THE INVENTION

The improved solid oral pharmaceutical composition of alendronic acid or the salt thereof of the present invention is characterized by physicochemical properties suitable for the tablet formulation by direct compression (dry granulation), the adequate release rate of the active ingredient and the storage stability, by employing excipients practically devoid of a tendency to interact with the active ingredient in a browning (Maillard) reaction, and possessing good compressibility properties.

It has been surprisingly found that the object of the present invention is achieved by employing Spray Dried Maltose of a grade specific for direct compression as a basic diluent. Although maltose is a reducing disaccharide, its reducing power is much lower than that of lactose and other reducing monosaccharides, and it does not undergo Maillard reaction with amines. It is a non-hygroscopic powder, more soluble than lactose.
Spray Dried Maltose is a more specific diluent for direct compression than Maltose. Specific tablet characteristics such as resistance to crashing and friability are much more improved with the use of Spray Dried Maltose over Maltose.This fact is justified by certain advantages of Spray Dried Maltose over Maltose which are: the improvement of the flow (angle of repose 25,00) and the tablet formulation properties by spray drying. Moreover, the spherical shape of its particles provides for excellent flow and has good disintegration by itself, improving also disintegration of other excipients such as Mannitol, Lactose and Cellulose. Furthermore, Spray Dried Maltose gives good compressibility and allows the decrease of the disintegrant content.

Therefore, in a first embodiment, the present invention provides a pharmaceutical composition comprising from about 1 to 50% by weight of Alendronate Sodium Trihydrate, and from about 50 to 99 % by weight of excipients, wherein said excipients comprise a basic diluent selected from Spray Dried Maltose, a disintegrant and a lubricant. More specifically, the disintegrant is Crospovidone and the lubricant is Calcium Stearate.

Preferred pharmaceutical compositions according to the present invention comprise approximately 1 to 50 % by weight of Alendronate Sodium Trihydrate; about 50 to 99 % by weight of Spray Dried Maltose; about 0.5 to 5 % by weight of Crospovidone; and about 0.1 to 2.5 % by weight of Calcium Stearate.

More preferred pharmaceutical compositions according to the present invention comprise approximately 3 to 50 % by weight of Alendronate Sodium Trihydrate; about 48 to 95 % by weight of Spray Dried Maltose; about 1 to 2 % by weight of Crospovidone; and about 0.5 to 1.5 % by weight of Calcium Stearate.

The preferred pharmaceutical compositions are in the form of solid dosage forms such as tablets, capsules, caplets, troches, pastilles, pills, lozenges and the like, in all shapes and sizes, coated or uncoated.
The preferred pharmaceutical compositions for commercial development include the following:

### Tablets of 10 mg (equivalent weight of Alendronic acid)

Approximately 6.53 % Alendronate Sodium Trihydrate; about 90.47 % Spray Dried Maltose or Spray Dried Mannitol; about 2.00 % Crospovidone and about 1.00 % Calcium Stearate.

### Tablets of 40 mg (equivalent weight of Alendronic acid)

Approximately 21.83% Alendronate Sodium Trihydrate; about 75.66% Spray Dried Maltose or Spray Dried Mannitol; about 1.67% Crospovidone and about 0.84% Calcium Stearate.

### Tablets of 70 mg (equivalent weight of Alendronic acid)

Approximately 32.83 % Alendronate Sodium Trihydrate; about 65.02 % Spray Dried Maltose or Spray Dried Mannitol; about 1.43 % Crospovidone and about 0.72 % Calcium Stearate.

Another embodiment of the present invention is the use of a dry granulation process for the preparation of solid dosage forms such as tablets containing Alendronate Sodium Trihydrate, which is one of the most economical methods. In a comparative study of development for the optimal process of manufacturing of Alendronate Sodium Trihydrate tablet compositions with Spray Dried Maltose by using either direct compression or dry granulation, it was found that the dry granulation process displays a number of specific advantages over the direct compression process. Namely, the dry granulation process results to an improved uniformity of mixture for tabletting, to a better fuction of the tabletting machine, and to an improved weight uniformity, resistance to crashing and friability of the tablets.

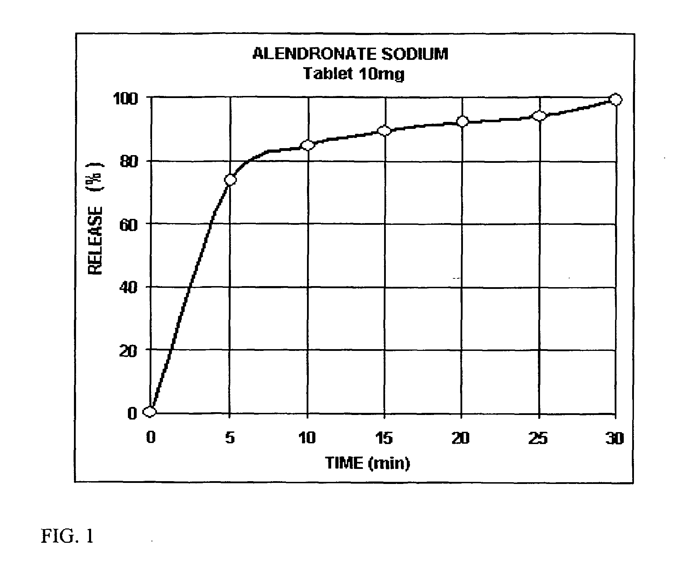

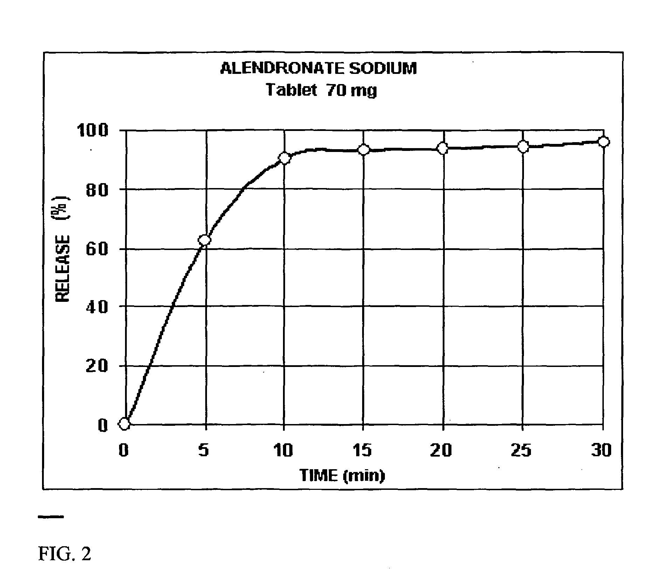

The dry granulation process of the present invention for the preparation of solid dosage forms such as tablets containing Alendronate Sodium Trihydrate as an active ingredient comprises:
- Weighing the required quantities of the active ingredient and all excipients;
- Forming a mixture by mixing the total quantity of the active ingredient with 50% of the quantity of a diluent selected from Spray Dried Maltose or Spray Dried Mannitol for 10 minutes;
- Slugging the above mixture;
- Screening the slugged mixture through a screen of 8 mesh, and subsequently through a screen of 20 mesh;
- Adding to the screened mixture the remaining 50% of the quantity of the Spray Dried Maltose, and the total quantities of a disintegrant such as Crospovidone and a lubricant such as Calcium Stearate and mixing for 30 minutes, and
- Tabletting, by compressing the resulting lubricated mixture into a desired tablet form.
The pharmaceutical compositions of the present invention are characterized by a small number of excipients and excellent pharmacotechnical properties, such as homogeneity, flowability and compressibility. Thanks to these properties, the solid dosage forms (tablets) prepared by the above process exhibit excellent technical characteristics including disintegration time, dissolution rate, hardness, resistance to crashing, friability and stability, as better illustrated by the following measurements during the stage of the development of the products.
Namely, the pure pharmaceutical substance showed acceptable flowability and compressibility with a Carr's Index between 35.3% and 37% (36% mean), Housner Ratio with a mean value of 1.56, whilst the angle of repose was found to be about 43°.
All the above results indicate that Alendronate Sodium Trihydrate has good to moderate flow properties, which can be improved by the addition of suitable ingredients in order to select a direct compression process for the final formulation.
The compressibility studies showed a good correlation (R²=0.95) with an exponential equation between resistance to crashing and compression force, indicating that the drug substance is suitable for a direct compression process in the presence of suitable excipients.
The pharmaceutical formulations according to the present invention have excellent pharmacotechnical properties indicating the suitability of the process and of the selected excipients as well.
Namely, a formulation composed of 6.5% Alendronate Sodium Trihydrate, 90.5% Spray Dried Maltose, 2% Crospovidone and 1% Calcium Stearate for the strength of 10mg (equivalent weight of alendronic acid per tablet) showed the following pharmacotechnical characteristics after slugging and compression in 500kp compression force, in round tablets 8mm diameter: Resistance to crashing: 8-11kp, Friability: 0.17%, Disintegration time: 3-4min, Dissolution: 98.7%. All the above characteristics are excellent for an immediate release product.
One of the most critical pharmacotechnical test, is the Dissolution test as it is strongly correlated with the bioavailability of the product. For the dissolution method an Apparatus II of European pharmacopoeia was used 50rpm, 37°C, time 30min, while as a dissolution medium 500ml of H₂O was used.
As shown in Fig. 1, the product displays a fast release profile, with more than 70% release in 5min, more than 80% in 15min, while the final release is about 100% in 30 minutes.
All the above mentioned characteristics were also investigated for a formulation of 70mg strength (equivalent weight of alendronic acid per tablet), which is composed of 21.8% Alendronate Sodium Trihydrate, 75.7% Spray Dried Maltose, 1.67% Crospovidone and 0.83% Calcium Stearate.
The same process of manufacturing as described above was used for the compression, and the tablet's characteristics were: Round biconvex tablets with a diameter of 11.2mm.
The results of the pharmacotechnical properties were the following: Resistance to crashing: 13.9kp, Friability: 0.14%, Disintegration time: 2min.
In order to prepare small, smoothly swallowable tablets the same mixture of excipients as for the strength of 10mg were used, namely Alendronate Sodium Trihydrate 32.83%, Maltose Spray Dried 65.02%, Crospovidone 1.43%, and Calcium Stearate 0.72%.
The same process was used and the following results were found: Resistance to crashing: 14.5kp, Friability: 0.12%, Disintegration time: 2.5min. The round biconvex punches, which were used, for the above trial corresponded to a diameter of 9.4mm.
As shown in Fig. 2, the dissolution profile of the composition of 70mg tablet showed a fast release profile with 62.5% release in 5min, while the release after 10 minutes was found to be more than 90%.
The most preferable compositions described below were investigated for their scalability, while a process validation was performed in order to prove the repeatability and accuracy of the manufacturing process and the proposed formulations. For the above tests 3 batches per strength were used.
The validation process showed that the compositions and the manufacturing process are suitable in order to provide a repeatable and high quality product.
Namely, the appearance was found to be acceptable in all cases. The resistance to crashing varied from 7kp to 12kp. The disintegration time was 2-5min, the dissolution was found to be over 90% in 30 minutes, whilst the Assay was between 98 and 102%.
No degradation products were observed during and after the procedure.
One of the main objects of the present invention was to prepare a product with acceptable stability and to avoid the browning caused by the Mailard's reaction. For this reason three batches of each strength (10mg and 70mg) were exposed to normal and accelerated stability studies according to the current ICH guidelines.
The following compositions were used per strength.

| INGREDIENT | STRENGTH 10mg | STRENGTH 70mg |
|---|---|---|
| Alendronate Sodium Trihydrate | 13.05mg | 91.37mg |
| equivalent to Alendronic Acid | 10.00mg | 70.00mg |
| Maltose (spray dried) | 180.95mg | 180.95mg |
| Crospovidone | 4.00mg | 4.00mg |
| Calcium Stearate | 2.00mg | 2.00mg |

The tablets were packed in PVC/PVDC-Aluminium Foils and stored in appropriate stability chambers at a temperature of 25°C±2°C and relative humidity of 60% ± 5% for normal conditions and at a temperature of 40°C and relative humidity of 75% for accelerated conditions. The tablets were tested in predetermined time intervals.

The frequency of the testing, the specific tests and results indicated for each batch are described in the stability table (TABLE 1).

**TABLE 1: STABILITY AT 25°C ± 2°C TEMPERATURE AND 60±5% RELATIVE HUMIDITY**

| PROPERTY* | | TIME IN MONTHS | | | | |
|---|---|---|---|---|---|---|
| | | 0 | 3 | 6 | 9 | 12 |
| Tablets 10 mg/tab | | | | | | |
| Appearance | | No change | No change | No change | No change | No change |
| Assay (by HPLC) | | 100.1% | 99.9% | 99.9% | 99.0% | 98.6% |
| Impurities: | Each known impurity: max 0.5% | <0.1 % | <0.1 % | <0.1 % | <0.1 % | <0.1 % |
| | Each unknown impurity: max 0.1 % | <0.1 % | <0.1 % | <0.1 % | <0.1 % | <0.1 % |
| Disintegration Time | | 2-4 min | 2 min | 2-2.5 min | 2.5 min | 2.5 min |
| Dissolution | | 100.5% | 99.0% | 99.3% | 98.3% | 98.7% |

| Tablets 70 mg/tab | | | | | | |
|---|---|---|---|---|---|---|
| Appearance | | No change | No change | No change | No change | No change |
| Assay (by HPLC) | | 101.2% | 101.5% | 101.5% | 100.4% | 100.0% |
| Impurities: | Each known impurity: max 0.5% | <0.1% | <0.1% | <0.1% | <0.1% | <0.1% |
| | Each unknown impurity: max 0.1 % | <0.1% | <0.1% | <0.1% | <0.1% | <0.1% |
| Disintegration Time | | 2.5-4 min | 2.5-3.5 min | 2.5-3 min | 2-2.5 min | 2-3 min |
| Dissolution | | 96.0-100.6% | 100.0% | 100.1% | 99.7% | 99.1% |

| | | | | | | |
|---|---|---|---|---|---|---|
| *Specifications Appearance: White round tablets Assay (by HPLC) : 90-110% during the shelf life Each known impurity: Max 0.5% Each unknown impurity: Max 0.1 % Disintegration Time: Max 15 minutes Dissolution : After 30 minutes: at least 85% of the labeled amount | | | | | | |

The results show a good stability of the product and compatibility between the drug substance and the excipients proposed by the present invention. The excellent results regarding the galenical and physicochemical characteristics, the excellent stability of the product as well as the simple and economic manufacturing process indicate the advantages of the present invention relative to the commonly used methods and excipients for the formulation of Alendronate Sodium Trihydrate.

The pharmaceutical compositions of the present invention may also contain one or more additional formulation ingredients selected from a wide variety of excipients. According to the desired properties of the composition, any number of ingredients may be selected, alone or in combination, based upon their known uses in preparation of solid dosage form compositions (tablet compositions). Such ingredients include, but are not limited to, diluents, compression aids, disintegrants, lubricants, flavors, sweetener, coating agents and preservatives. The pharmaceutical compositions of the present invention do not, however, require the addition of a separate dry binder.

The following examples illustrate preferred embodiments in accordance with the present invention without limiting the scope or spirit of the invention.

### EXAMPLES

### Example 1:

### Tablet of 10 mg Alendronic acid (equivalent weight)

### Maltose formula

| Ingredients | Per Tablet |
|---|---|
| Alendronate Sodium Thihydrate equivalent to 10.00 mg Alendronic Acid | 13.05 mg |
| Maltose (spray dried) | 180.95 mg |
| Crospovidone | 4.00 mg |
| Calcium Stearate | 2.00 mg |
| Total weight: | 200.00 mg |

The manufacturing process comprises the following steps:
1. Weighing
2. Screening all the materials
3. Blending for 10 minutes the following ingedients
   - Alendronate Sodium Trihydrate 100%
   - Spray Dried Maltose 50%
   Due to the low quantity of the active ingredient, this mixture has a better uniformity of content in the final mixture prior the tablet formulation.
4. Slugging
   Slugging the above mixture and then screening through a 8 mesh screen and subsequently through a 20 mesh screen.
5. Blend for 30 to 45 minutes
   - Screened Mixture 100%
   - Spray Dried Maltose 50%
   - Crospovidone 100%
   - Calcium Stearate 100%
6. Tabletting
   Compressing this mixture in a Manesty tabletting machine with round punches.
7. Packaging

The tablets are packed into blisters of PVC-PVDC/Aluminium foil.

### Example 2 :

### Tablet of 40 mg Alendronic acid (equivalent weight)

### Maltose formula

| Ingredients | Per Tablet |
|---|---|
| Alendronate Sodium Thihydrate equivalent to 40.00 mg Alendronic Acid | 52.20 mg |
| Maltose (spray dried) | 180.95 mg |
| Crospovidone | 4.00 mg |
| Calcium Stearate | 2.00 mg |
| Total weight: | 239.15 mg |

Tablets were prepared using the procedure of Example 1.

### Example 3

### Tablet of 70 mg Alendronic acid (equivalent weight)

### Maltose formula

| Ingredients | Per Tablet |
|---|---|
| Alendronate Sodium Thihydrate equivalent to 70.00 mg Alendronic Acid | 91.37 mg |
| Maltose (spray dried) | 180.95 mg |
| Crospovidone | 4.00 mg |
| Calcium Stearate | 2.00 mg |
| Total weight: | 278.32 mg |

Tablets were prepared using the procedure of Example 1.

### Example 4

### Tablet of 10 mg Alendronic acid (equivalent weight)

### Mannitol formula

| Ingredients | Per Tablet |
|---|---|
| Alendronate Sodium Thihydrate equivalent to 10.00 mg Alendronic Acid | 13.05 mg |
| Mannitol (spray dried) | 180.95 mg |
| Crospovidone | 4.00 mg |
| Calcium Stearate | 2.00 mg |
| Total weight: | 200.00 mg |

Tablets were prepared using the procedure of Example 1.

## Claims

1. A pharmaceutical composition comprising from about 1 to 50% by weight of Alendronate Sodium Trihydrate, as an active ingredient, and from 50 to 99 % by weight of excipients, wherein said excipients comprise the basic diluent Spray Dried Maltose, a disintegrant and a lubricant.

2. The pharmaceutical composition according to claim 1, wherein said disintegrant is Crospovidone.

3. The pharmaceutical composition according to claim 1, wherein said lubricant is Calcium Stearate.

4. The pharmaceutical composition according to claim 1, comprising: about 1 to 50 % by weight of Alendronate Sodium Trihydrate; about 50 to 99 % by weight of Spray Dried Maltose; about 0.5 to 5 % by weight of Crospovidone; and about 0.1 to 2.5 % by weight of Calcium Stearate.

5. The pharmaceutical composition according to claim 1, comprising: about 3 to 50 % by weight of Alendronate Sodium Trihydrate; about 48 to 95 % by weight of Spray Dried Maltose; about 1 to 2 % by weight of Crospovidone; and about 0.5 to 1.5 % by weight of Calcium Stearate.

6. The pharmaceutical composition according to any preceding claim, wherein said composition is in a solid dosage form such as a tablet comprisig an active ingredient such as Alendronate Sodium Trihydrate equivalent to 10.00mg or 40.00mg or 70.00mg Alendronic acid and Spray Dried Maltose as a basic diluent.

7. A tablet prepared from the pharmaceutical composition according to any preceding claim.

8. A process for the preparation of a solid dosage form, such as a tablet, containing an active ingredient alendronic acid or the salt thereof , which comprises:
- Weighing the required quantities of the active ingredient and of all excipients;
- Forming a mixture by mixing the total quantity of the active ingredient with 50% of the quantity of a diluent selected from Spray Dried Maltose for 10 minutes;
- Slugging the above mixture;
- Screening the slugged mixture through a screen of 8 mesh, and subsequently through a screen of 20 mesh;
- Adding to the screened mixture the remaining 50% of the quantity of the Spray Dried Maltose, and the total quantities of a disintegrant and a lubricant and mixing for 30 minutes, and
- Tabletting, by compressing the resulting lubricated mixture into a desired tablet form.

9. The process according to claim 8, wherein said active ingredient is alendronate sodium trihydrate.

10. The process according to claim 8, wherein said disintegrant is Crospovidone.

11. The process according to claim 8, wherein said lubricant is Calcium Stearate.

## Patentansprüche

1. Pharmazeutische Formulierung, die aus etwa 1 bis 50 Gew.-% Alendronat Natrium Trihydrat als Wirkstoff und etwa 50 bis 99 Gew.-% Exzipiente enthält, wobei die Exzipiente als grundlegendes Verdünnungsmittel das sprühgetrockneter Maltose beinhalten sowie ein Sprengmittel und ein Gleitmittel.

2. Pharmazeutische Formulierung nach Ansprüch 1, wobei das Sprengmittel Crospovidone ist.

3. Pharmazeutische Formulierung nach Ansprüch 1, wobei das Gleitmittel Calciumstearat ist.

4. Pharmazeutische Formulierung nach Ansprüch 1, die aus etwa 1 bis 50 Gew.-% Alendronat Natrium Trihydrat, etwa 50 bis 99 Gew.-% sprühgetrockneter Maltose, etwa 0,5 bis 5 Gew.-% Crospovidone und etwa 0,1 bis 2,5 Gew.-% Calciumstearat besteht.

5. Pharmazeutische Formulierung nach Ansprüch 1, die aus etwa 3 bis 50 Gew.-% Alendronat Natrium Trihydrate, etwa 48 bis 95 Gew.-% sprühgetrockneter Maltose, 1 bis 2 Gew.-% Crospovidone und 0,5 bis 1,5 Gew.-% Calciumstearat besteht.

6. Pharmazeutische Formulierung nach einem vorausgegangenen Ansprüch, wobei die Formulierung eine feste Arzneiform wie eine Tablette ist, die aus ein Wirkstoff wie Alendronat Natrium Trihydrat, das 10 mg oder 40 mg oder 70 mg Alendronsäure entspricht, und sprühgetrockneter Maltose als grundlegendes Verdünnungsmittel besteht.

7. Tablette hergestellt entsprechend der pharmazeutischen Formulierung nach einem vorausgegangenen Ansprüch.

8. Verfahren zur Herstellung eines festen Arzneimittels wie einer Tablette, welche den Wirkstoff Alendronsäure oder dessen Salz enthält und das umfasst:
- Abwiegen der erforderlichen Quantitäten der aktiven Inhaltsstoffe und aller sonstigen Bestandteile;
- Herstellung einer Mixtur durch die Vermischung der gesamten Menge der Wirkstoffe mit 50 % der Gleitmittel aus sprühgetrockneter Maltose für 10 Minuten;
- Schlagen der oben genannten Mixtur;
- Die geschlagene Mixtur durch ein Sieb von 8-Sieblochung und anschließend durch ein Sieb von 20 Sieblochung geben;
- Der gesiebten Mixtur die verbleibenden 50 % der sprühgetrockneten Maltose sowie die gesamten Mengen des Sprengmittels und des Gleitmittels hinzufügen und 30 Minuten vermischen und
- Tablettenform herstellen durch das Komprimieren der Gleitmittelmasse in die gewünschte Tablettenform.

9. Verfahren nach Ansprüch 8, wobei der Wirkstoffe Alendronate Natrium Trihydrat ist.

10. Verfahren nach Ansprüch 8, wobei das Sprengmittel Crospovidone ist.

11. Verfahren nach Ansprüch 8, wobei das Gleitmittel Calciumstearat ist.

## Revendications

1. Préparation pharmaceutique comprenant entre environ de 1 à 50% en poids d'alendronate sodium trihydrate, comme substance active, et de 50 à 99% en poids d'excipients, lesdits excipients comprenant comme diluant principale le maltose séché par pulvérisation, ,un agent désintégrant et un agent lubrifiant

2. Préparation pharmaceutique selon la revendication 1, dans laquelle l'agent désintégrant est la crospovidone

3. Préparation pharmaceutique selon la revendication 1, dans laquelle l'agent lubrifiant est le stéarate de calcium.

4. Préparation pharmaceutique selon la revendication 1, comprenant environ de 1 à 50% en poids d'alendronate sodium trihydrate, environ de 50 à 90% en poids de maltose séché par pulvérisation, environ de 0.5 à 5% en poids de crospovidone, et environ de 0.1 à 2.5% en poids de stéarate de calcium.

5. Préparation pharmaceutique selon la revendication 1, comprenant : environ de 3 à 50% en poids d'alendronate sodium trihydrate, environ de 48 à 95% en poids de maltose séché par pulvérisation, environ de 1 à 2% en poids de cropovidone, et environ de 0.1 à 1.5% en poids de stéarate de calcium.

6. Préparation pharmaceutique selon une des revendications précédentes, dans lesquelles ladite préparation est en forme solide comme des comprimes contenant une substance active comme le sodium trihydrate d'alendronate équivalent à 10mg ou 40mg ou 70mg d'acide alendronique et le maltose séché par pulvérisation comme diluent principal.

7. Comprimé préparé à partir de la préparation pharmaceutique selon une des revendications précédentes.

8. Procédé de fabrication d'une unité de dosage de forme solide, comme un comprimé, contenant comme substance active l'acide alendronique or un des ses sels, ledit procédé comprenant les étapes suivantes :
a. Pesée des quantités requises de la substance active et de tous les excipients ;
b. Création d'un mélange par le mélange de la totalité de la substance active avec 50% de la quantité du diluant maltose séché par pulvérisation pendant 10minutes ;
c. Pré-compression en chapelure du mélange ci-dessus
d. Filtration de la chapelure à travers un tamis de 8 mesh suivi d'un tamis de 20 mesh
e. Ajout au filtrat des 50% restant de la quantité du maltose séché par pulvérisation ainsi que de la totalité des quantités de l'agent désintégrant et de l'agent lubrifiant suivi d'un mélange pendant 30 minutes et,
f. Compression du mélange lubrifié résultant en comprimés selon la forme désirée.

9. Le procédé selon la revendication 8, dans lequel la substance active est l'alendronate de sodium trihydrate.

10. Le procédé selon la revendication 8, selon lequel l'agent désintégrant est la crospovidone.

11. Le procédé selon la revendication 8, selon laquelle l'agent lubrifiant est le stéarate de calcium.
